# EUROPÄISCHE PATENTSCHRIFT

(11) **EP 1 624 830 B1**
(45) Veröffentlichungstag und Bekanntmachung des Hinweises auf die Patenterteilung: **28.05.2008**
(21) Anmeldenummer: 04726859.4
(22) Anmeldetag: 10.04.2004
(51) Int. Cl.: A61F 2/44, A61F 2/30

(54) **WIRBELSÄULENIMPLANTAT**
VERTEBRAL COLUMN IMPLANT
IMPLANT DE COLONNE VERTEBRALE

(30) Priorität: 21.05.2003 DE 10324108; 24.07.2003 DE 10333659
(43) Veröffentlichungstag der Anmeldung: 15.02.2006
(73) Patentinhaber: AESCULAP AG & Co. KG, 78532 Tuttlingen (DE)
(72) Erfinder: LINDNER, Stephan, 78532 Tuttlingen (DE); SCHUMACHER, Jörg, 78532 Tuttlingen (DE); KRAMER, Ulrich, 8374 Oberwangen (CH); BADER, Uwe, 78532 Tuttlingen (DE); WALLSTEIN, Stefan, 78576 Emmingen-Liptingen (DE); SCHULTZ, Robert, 78532 Tuttlingen (DE); BEGER, Jens, 78532 Tuttlingen (DE)
(74) Vertreter: Boehme, Ulrich
(86) Internationale Anmeldenummer: PCT/EP2004/003831
(87) Internationale Veröffentlichungsnummer: WO 2004/103226

(56) Entgegenhaltungen:
- DE-C- 19 816 832
- US-B1- 6 193 757
- US-B1- 6 206 923

## Beschreibung

Die Erfindung betrifft ein Wirbelsäulenimplantat mit einer eine Anlagefläche zur Abstützung an einem Wirbelkörper bildenden Anlageplatte.

Wirbelsäulenimplantate können eingesetzt werden in einem Zwischenwirbelraum, um eine entfernte Bandscheibe zu ersetzen und somit zwei unmittelbar benachbarte Wirbelkörper gegeneinander abzustützen, sei es durch eine starre Verbindung, sei es durch eine Gelenkverbindung. In diese Falle liegen die Wirbelsäulenimplantate als flache Implantate mit ihren Anlageflächen an den Stirnseiten benachbarter Wirbelkörper an.

Wirbelsäulenimplantate dieser Art werden aber auch als Wirbelkörperersatzimplantate benötigt, die einen oder mehrere fehlende Wirbelkörper überbrücken sollen. Derartige Implantate haben dann eine erhebliche Höhe, da sie mindestens so hoch sein müssen wie ein Wirbelkörper, damit unterscheiden sich derartige Wirbelkörperersatzimplantate deutlich von Zwischenwirbelimplantaten, die als Bandscheibenersatz oder als Fusionsimplantat zwischen zwei Wirbelkörper eingeschoben werden, die natürlich unmittelbar nebeneinanderliegen.

Um eine zuverlässige Abstützung des Wirbelsäulenimplantates an den benachbarten Wirbelkörpern zu gewährleisten, ist es sowohl bei Wirbelkörperersatzimplantaten als auch bei Zwischenwirbelimplantaten günstig, möglichst große Anlageflächen zu verwenden, damit sich die Druckkräfte großflächig verteilen und Druckspitzen vermieden werden. Die Anlageflächen entsprechen somit häufig der Fläche der Wirbelkörperendflächen, und daher kann es schwierig sein, diese Wirbelsäulenimplantate in den Körper einzuführen. Es sind dazu Zugänge mit großem Durchmesser notwendig. Dies verhindert minimalinvasive Zugänge und erschwert auch die Durchführung des Implantates zwischen Knochenteilen des Skeletts, beispielsweise zwischen Rippenbögen.

Dasselbe Problem ergibt sich bei Implantaten, die zwar nicht einen vollständig fehlenden Wirbelkörper ersetzen sollen, die aber einen geschwächten oder teilweise fehlenden Wirbelkörper verstärken sollen, beispielsweise dann, wenn dieser Wirbelkörper aufgrund von Osteoporose Frakturen zeigt. In diesen Fällen können derartige Implantate seitlich durch eine in den Wirbelkörper eingebrachte Öffnung in diesen eingeführt werden und durchsetzen dann diesen zu verstärkenden Wirbelkörper, wobei die Anlageflächen des Implantates wie bei einem Wirbelkörperersatzimplantat an den Anlageflächen der Wirbelkörper anliegen, die zu beiden Seiten an den zu verstärkenden Wirbelkörper angrenzen. In diesem Falle ist das Implantat also ein Wirbelkörperunterstützungsimplantat, dessen Länge aber natürlich ebenso wie bei einem Wirbelkörperersatzimplantat den gesamten Abstand zwischen den Wirbelkörpern überbrücken muss, die zu beiden Seiten an den geschwächten und zu verstärkenden Wirbelkörper angrenzen. Aus diesem Grunde wird nachstehend für alle Implantate dieser Art der gemeinsame Ausdruck Wirbelsäulenimplantat verwendet, auch wenn es sich im Einzelfalle um ein Implantat handelt, das einen noch vorhandenen Wirbelkörper durchsetzt und ihn dadurch unterstützt.

In der US-B1-6,193,757 ist ein Wirbelsäulenimplantat beschrieben, bei dem stegförmige Stützflächen durch Verschwenkung von Implantatteilen oder durch Verschiebung von zwei Implantatteilen gegeneinander in ihrem Abstand verändert werden können, so dass das Wirbelsäulenimplantat zum Einführen in den Körper eine kleinere Kontur aufweist als im eingesetzten Zustand, bei dem die stegförmigen Stützflächen einen größeren Abstand voneinander aufweisen und daher die Wirbelfläche in weiter außen liegenden, stabileren Bereichen unterstützen können. Die Unterstützung erfolgt dabei aber nur in stegförmigen Bereichen, so dass unter Umständen relativ hohe Druckspitzen auftreten können. Außerdem weist dieses Implantat scharfe Kanten auf, die das Einführen schwierig gestalten können.

Es ist Aufgabe der Erfindung, ein Wirbelsäulenimplantat der eingangs beschriebenen Art so auszugestalten, dass es auch durch Zugänge mit kleinerem Durchmesser problemlos in den Körper eingeführt werden kann und dass trotzdem die Möglichkeit besteht, dass das Wirbelsäulenimplantat einen angrenzenden Wirbelkörper großflächig abstützt.

Diese Aufgabe wird bei einem Wirbelsäulenimplantat der eingangs beschriebenen Art gemäß einer ersten bevorzugten Ausführungsform erfindungsgemäß dadurch gelöst, dass die Anlageplatte eine Vertiefung aufweist, die mindestens einen Stützarm aufnimmt, der um eine senkrecht auf der Anlageplatte stehende Schwenkachse verschwenkbar an der Anlageplatte gelagert ist, und dass der mindestens eine schwenkbare Stützarm zwischen einem eingeschwenkten Zustand, in dem die Anlageplatte den mindestens einen Stützarm vollständig überdeckt, und einem ausgeschwenkten Zustand verschwenkbar ist, in dem der mindestens eine Stützarm über die Außenkontur der Anlageplatte hervorragt.

Es wird also dafür Sorge getragen, dass die Anlagefläche zwar in der Anlageposition, in der sie an der Wirbelkörperendfläche anliegt und in der sie endgültig implantiert ist, die volle Ausdehnung aufweist und damit eine gute Druckverteilung garantiert, es wird aber durch Änderung der Form oder der Anordnung dieser Anlagefläche erreicht, dass für die Einführung des Implantates in den Körper die Ausdehnung der Anlagefläche in ihrer Ebene geringer ist als in der endgültigen Anlageposition. Dadurch ergibt sich für das gesamte Implantat eine geringere Abmessung, und dies erleichtert das Einführen in den Körper durch einen Zugang mit kleinerem Durchmesser.

Die Schwenkachse des Stützarms kann dabei an einer Längskante der Anlageplatte angeordnet sein, insbesondere im Eckbereich.

Vorteilhaft ist es, wenn an gegenüberliegenden Seitenkanten der Anlageplatte je ein Stützarm gelagert ist.

Der Stützarm kann in der ausgeschwenkten Stellung durch eine Arretierung in dieser Stellung fixiert und dadurch gegen ein Einschwenken gesichert sein, beispielsweise kann die Arretierung eine sich beim Ausschwenken vom Stützarm wegbiegende Blattfeder sein.

Bei einer zweiten Ausführungsform wird diese Aufgabe bei einem Wirbelsäulenimplantat der eingangs beschriebenen Art erfindungsgemäß dadurch gelöst, dass die Anlageplatte eine Vertiefung aufweist, die einen in einer Führung parallel zu sich selbst verschiebbaren Stützkörper aufnimmt, der zwischen einer eingeschobenen Stellung, in welcher die Anlageplatte den Stützkörper vollständig überdeckt, in eine ausgeschobene Stellung verschiebbar ist, in der der Stützkörper über die Außenkontur der Anlageplatte hervorragt.

Dieser Stützkörper kann insbesondere U-förmig ausgebildet sein und parallel zu seinen Schenkeln verschiebbar sein.

Auch hier ist es vorteilhaft, wenn der Stützkörper in der ausgeschobenen Stellung durch eine Arretierung in dieser Stellung fixiert und dadurch gegen ein Einschieben gesichert ist.

Die Länge der Anlageplatte kann etwa doppelt so groß sein wie deren Breite, so dass ein besonders günstiges Einführen in den Körper möglich ist.

Die Anlageplatte kann auf ihrer dem Wirbelkörper abgewandten Seite über ein Gelenk an einer weiteren Anlageplatte schwenkbar abgestützt sein, dies ist insbesondere bei Implantaten günstig, die als Zwischenwirbelimplantat eingesetzt werden.

Das Gelenk umfasst dabei gemäß einer bevorzugten Ausführungsform zusammenwirkende ballige Lagerflächen aus Keramik.

Die nachfolgende Beschreibung bevorzugter Ausführungsform der Erfindung dient im Zusammenhang mit der Zeichnung der näheren Erläuterung. Es zeigen:
- Figur 1:: eine perspektivische Ansicht einer Anlageplatte eines Zwischenwirbelimplantates mit eingeschwenkten Stützarmen;
- Figur 2:: eine Seitenansicht eines Zwischenwirbelimplantates mit zwei Anlageplatten;
- Figur 3:: eine Schnittansicht längs Linie 3-3 in Figur 2;
- Figur 4:: eine Draufsicht auf die Anlageplatte der Figur 1 mit einem eingeschwenkten und mit einem ausgeschwenkten Stützarm;
- Figur 5:: eine Ansicht ähnlich Figur 4 bei einem anderen bevorzugten Ausführungsbeispiel einer Anlageplatte mit einem Stützarm;
- Figur 6:: eine Seitenansicht eines abgewandelten Ausführungsbeispiels einer Anlageplatte eines Zwischenwirbelimplantates mit einem ausziehbaren Stützelement und
- Figur 7:: eine Draufsicht auf die Anlageplatte der Figur 6 mit dem Stützelement in unterschiedlichen Stellungen.

In der Zeichnung sind Zwischenwirbelimplantate 41 dargestellt, die nach der Entfernung der Bandscheibe in den Zwischenwirbelraum zwischen zwei benachbarten Wirbelkörpern eingesetzt werden können.

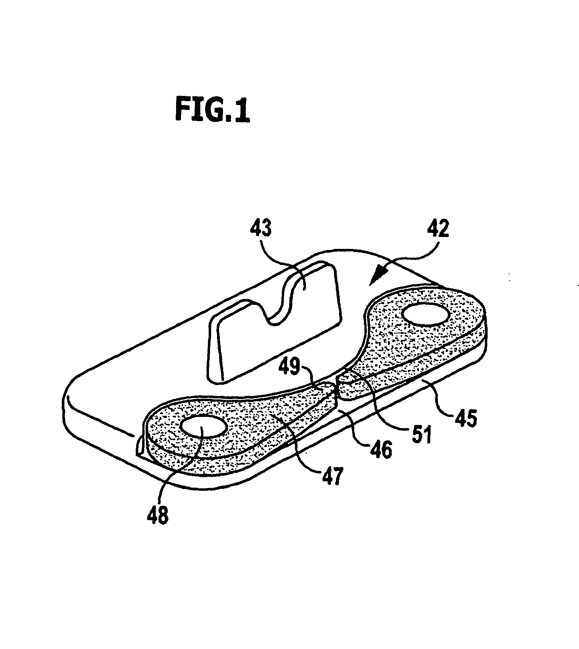

Das in den Figuren 1 bis 4 dargestellte Zwischenwirbelimplantat 41 umfasst eine etwa rechteckförmige längliche Anlageplatte 42, die etwa doppelt so lang wie breit ist. Die Länge entspricht dabei etwa der Querabmessung der Wirbelkörperstützfläche, quer dazu ist die Anlageplatte 42 dagegen nur etwa halb so breit wie die Wirbelkörperanlagefläche. Die Form ist in dem dargestellten Ausführungsbeispiel im wesentlichen rechteckig gewählt, es könnte auch eine nierenförmige oder in anderer Weise gebogene Form gewählt werden, die an die Kontur der Wirbelkörperfläche angepasst ist.

Auf einer Seite trägt die Anlageplatte 42 Verankerungsvorsprünge 43, die bei Anlage an einem Wirbelkörper in diesen eindringen und die Anlageplatte 42 festlegen.

Zwei derartige Anlageplatten 42 bilden gemeinsam ein Zwischenwirbelimplantat 41 aus, dazu sind beide Anlageplatten 42 auf den einander zugewandten Seiten mit balligen, komplementär zueinander ausgebildeten und flächig aneinander anliegenden Gelenkflächen 44 ausgestattet, die beispielsweise durch keramische Einsatzkörper gebildet werden, die in entsprechende Ausnehmungen der Anlageplatte 42 fest eingesetzt sind. Dadurch stützen sich die beiden Anlageplatten 42 schwenkbar aneinander ab und können in gewissen Grenzen gegeneinander verschwenkt werden.

Jede der beiden Anlageplatten 42 weist längs einer Längskante 45 eine Vertiefung 46 auf, die jeweils zwei Stützarme 47 aufnehmen. Bei dem in den Figuren 1 bis 4 dargestellten Ausführungsbeispiel sind beide Stützarme 47 spiegelbildlich zueinander aufgebaut, es wird daher nur einer der Stützarme 47 näher erläutert. Der Stützarm ist um eine senkrecht auf der Anlageplatte 42 stehende Schwenkachse schwenkbar an dieser gelagert, die Schwenkachse befindet sich in einem Eckbereich. In diesem Bereich umgibt der Stützarm 47 die Schwenkachse 48 ösenförmig und erstreckt sich mit einem Verlängerungsteil 49 etwa bis zur Mitte der Anlageplatte 42, wenn beide Stützarme 47 in die Vertiefung 46 eingeschwenkt sind (Figur 1). Die Verlängerungsteile 49 liegen dann in der Mitte der Anlageplatte 42 unmittelbar einander gegenüber, die Stützarme 47 werden dabei von der Anlageplatte 42 vollständig überdeckt.

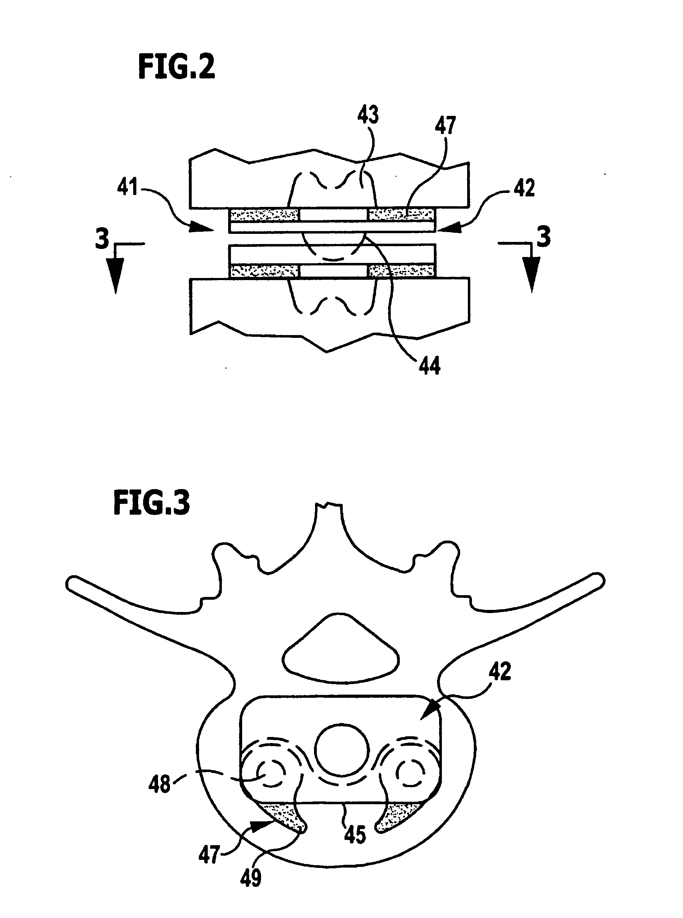

Beide Stützarme 47 sind aus dieser Lage ausschwenkbar, so dass das Verlängerungsteil 49 über die Außenkontur der Anlageplatte 42 hervorragt und dadurch die effektive Anlagefläche der Anlageplatte 42 vergrößert (Figur 3). In den Stützarm 47 ist seitlich eine Blattfeder 50 eingelegt, die beim Ausschwenken des Stützarms 47 ausfedert und sich mit ihrem freien Ende an einer Kante 51 der Vertiefung 46 so anlegt, dass der Stützarm 47 nicht mehr in die eingeschwenkte Stellung zurückgeschwenkt werden kann. Man erhält damit eine Sicherung gegen ungewolltes Einschwenken des Stützarmes 47.

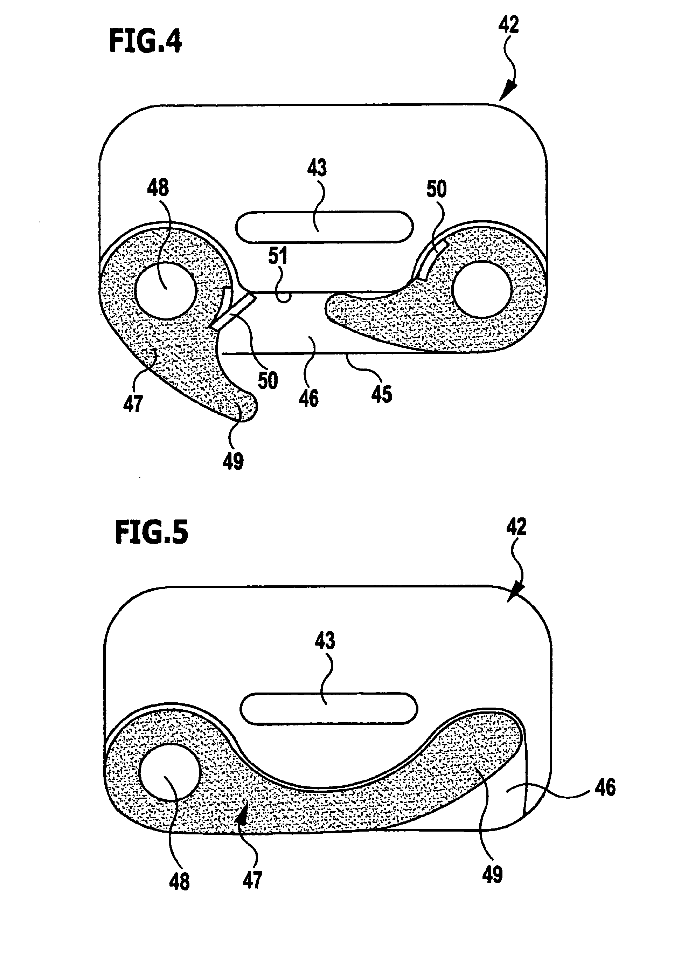

Bei dem Ausführungsbeispiel der Figur 5, das im wesentlichen gleich aufgebaut ist und bei dem daher gleiche Teile dieselben Bezugszeichen tragen, sind lediglich die zwei Stützarme 47 ersetzt durch einen einzigen Stützarm 47, der sich im wesentlichen über die gesamte Länge der Anlageplatte 42 erstreckt. Das Verlängerungsteil 49 ist dabei bogenförmig ausgebildet, so dass beim Ausschwenken des Stützarmes 47 eine möglichst starke Vergrößerung der effektiven Anlagefläche erfolgt und sich das Verlängerungsteil 49 nach Möglichkeit über den Randbereich des Wirbelkörpers erstreckt, der eine besonders hohe Festigkeit aufweist. Dies gilt im übrigen auch bei den Stützarmen 47 beim Ausführungsbeispiel der Figuren 1 bis 4, auch hier reichen die Verlängerungsteile 49 in den besonders stabilen Randbereich des Wirbelkörpers und stützen daher die Anlageplatte besonders wirksam am Wirbelkörper ab.

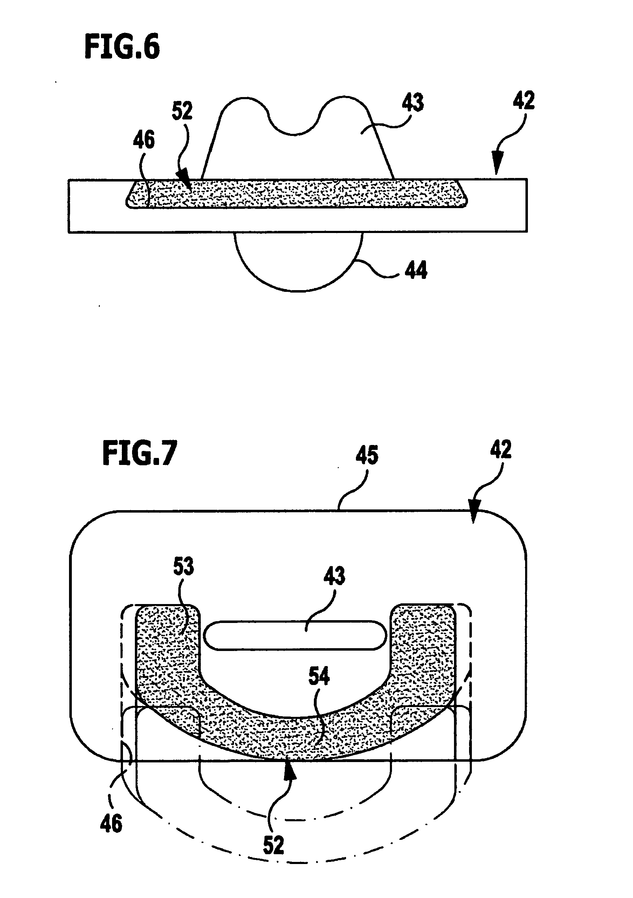

Während bei den Ausführungsbeispielen der Figuren 1 bis 5 zur Vergrößerung der effektiven Anlagefläche der Anlageplatte 42 verschwenkbare Stützarme verwendet werden, weist die Anlageplatte 42 des Ausführungsbeispiels der Figuren 6 und 7, das im übrigen ähnlich aufgebaut ist und bei dem gleiche Teile dieselben Bezugszeichen tragen, ein Stützelement 52 auf, welches U-förmig ausgebildet ist und somit zwei parallele Schenkel 53 und einen diese verbindenden, gebogenen Steg 54 aufweist. Dieses Stützelement 52 ist parallel zu seinen Schenkeln 53 verschieblich in der Vertiefung 46 gelagert und kann somit aus einer eingeschobenen Position, in welcher die Anlageplatte 42 das Stützelement 52 vollständig überdeckt (in Figur 7 mit ausgezogenen Linien gezeichnet) in eine ausgeschobene Stellung verschoben werden (in Figur 7 in strichpunktierten Linien), in welcher der Steg 54 und Teile der Schenkel 53 über die Kontur der Anlageplatte 42 hervorstehen und somit deren effektive Anlagefläche vergrößern. Auch in diesem Falle kann eine Arretierung vorgesehen sein, beispielsweise unter Verwendung einer Blattfeder, wie dies am Beispiel der Figur 4 gezeigt ist.

Durch die relativ kleinen Abmessungen der Anlageplatte 42 ist es möglich, dieses Zwischenwirbelimplantat von der Seite her in den Zwischenwirbelraum einzuführen und nicht, wie das sonst üblich ist, ventral, dadurch kann eine Implantation auch in Fällen erfolgen, in denen aufgrund anatomischer Gegebenheiten die ventrale Einführung Schwierigkeiten bereiten würde oder unmöglich wäre. Trotzdem kann durch Ausschwenken oder Ausziehen der Stützarme bzw. Stützelemente die effektive Anlagefläche der Anlageplatte 42 so weit vergrößert werden, dass die Stützkräfte auf eine sehr große Anlagefläche verteilt werden, so dass keine Gefahr besteht, dass die Anlageplatten 42 in die Wirbelkörper einbrechen.

Die verschiedenen Konstruktionen zur Vergrößerung der Anlageflächen können wahlweise zwischen Wirbelkörperersatzimplantaten und Zwischenwirbelimplantaten ausgetauscht werden, d.h. die in den Beispielen anhand von Zwischenwirbelimplantaten und Wirbelkörperersatzimplantaten beschriebenen Konstruktionen sind nicht auf diese allein beschränkt.

## Patentansprüche

1. Wirbelsäulenimplantat (1) mit einer eine Anlagefläche zur Abstützung an einem Wirbelkörper bildenden Anlageplatte (42), **dadurch gekennzeichnet, dass** sie eine Vertiefung (46) aufweist, die mindestens einen Stützarm (47) aufnimmt, der um eine senkrecht auf der Anlageplatte (42) stehende Schwenkachse (48) verschwenkbar an der Anlageplatte (42) gelagert ist, und dass der mindestens eine schwenkbare Stützarm (47) zwischen einem eingeschwenkten Zustand, in dem die Anlageplatte (42) den mindestens einen Stützarm (47) vollständig überdeckt, und einem ausgeschwenkten Zustand verschwenkbar ist, in dem der mindestens eine Stützarm (47) über die Außenkontur der Anlageplatte (42) hervorragt.

2. Wirbelsäulenimplantat nach Anspruch 1, **dadurch gekennzeichnet, dass** die Schwenkachse (48) des Stützarms (47) an einer Längskante (45) der Anlageplatte (42) angeordnet ist.

3. Wirbelsäulenimplantat nach Anspruch 1 oder 2, **dadurch gekennzeichnet, dass** an gegenüberliegenden Seitenkanten der Anlageplatte (42) je ein Stützarm (47) gelagert ist.

4. Wirbelsäulenimplantat nach einem der voranstehenden Ansprüche, **dadurch gekennzeichnet, dass** der Stützarm (47) in der ausgeschwenkten Stellung durch eine Arretierung (50) in dieser Stellung fixiert und **dadurch** gegen ein Einschwenken gesichert ist.

5. Wirbelsäulenimplantat nach Anspruch 4, **dadurch gekennzeichnet, dass** die Arretierung eine sich beim Ausschwenken vom Stützarm (47) weg biegende Blattfeder (50) ist.

6. Wirbelsäulenimplantat mit einer eine Anlagefläche zur Abstützung an einem Wirbelkörper bildenden Anlageplatte (42), **dadurch gekennzeichnet, dass** sie eine Vertiefung (46) aufweist, die einen in einer Führung parallel zu sich selbst verschiebbaren Stützkörper (52) aufnimmt, der zwischen einer eingeschobenen Stellung, in welcher die Anlageplatte (42) den Stützkörper (52) vollständig überdeckt, in eine ausgeschobene Stellung verschiebbar ist, in der der Stützkörper (52) über die Außenkontur der Anlageplatte (42) hervorragt.

7. Wirbelsäulenimplantat nach Anspruch 6, **dadurch gekennzeichnet, dass** der Stützkörper (52) U-förmig ausgebildet und parallel zu seinen Schenkeln (53) verschiebbar ist.

8. Wirbelsäulenimplantat nach Anspruch 6 oder 7, **dadurch gekennzeichnet, dass** der Stützkörper (52) in der ausgeschobenen Stellung durch eine Arretierung in dieser Stellung fixiert und **dadurch** gegen ein Einschieben gesichert ist.

9. Wirbelsäulenimplantat nach einem der voranstehenden Ansprüche, **dadurch gekennzeichnet, dass** die Länge der Anlageplatte (42) etwa doppelt so groß ist wie deren Breite.

10. Wirbelsäulenimplantat nach einem der voranstehenden Ansprüche, **dadurch gekennzeichnet, dass** die Anlageplatte (42) auf ihrer dem Wirbelkörper abgewandten Seite über ein Gelenk (44) an einer weiteren Anlageplatte (42) schwenkbar abgestützt ist.

11. Wirbelsäulenimplantat nach Anspruch 10, **dadurch gekennzeichnet, dass** das Gelenk zusammenwirkende ballige Lagerflächen (44) aus Keramik umfasst.

## Claims

1. A vertebral column implant (1) having a contact plate (42) forming a contact surface for support on a vertebral body, **characterised in that** the plate has a depression (46) receiving at least one support arm (47) mounted on the contact plate (42) so as to be pivotable about a pivot axis (48) perpendicular to the contact plate (42), and **in that** the at least one pivotable support arm (47) is pivotable between a swung in state in which the contact plate (42) completely covers the at least one support arm (47) and a swung out state in which the at least one support arm (47) projects beyond the outer contour of the contact plate (42).

2. A vertebral column implant according to claim 1, **characterised in that** the pivot axis (48) of the support arm (47) is arranged on a longitudinal edge (45) of the contact plate (42).

3. A vertebral column implant according to claim 1 or 2, **characterised in that** a respective support arm (47) is mounted on facing lateral edges of the contact plate (42).

4. A vertebral column implant according to any one of the preceding claims, **characterised in that** the support arm (47) in the swung out position is fixed in this position by a locking means (50) and is thereby secured against swinging in.

5. A vertebral column implant according to claim 4, **characterised in that** the locking means is a leaf spring (50) bending away from the support arm (47) upon swinging out.

6. A vertebral column implant having a contact plate (42) forming a contact surface for support on a vertebral body, **characterised in that** the plate has a depression (46) receiving a support body (52) displaceable parallel to itself in a guide, which support body (52) is displaceable between a pushed in position in which the contact plate (42) completely covers the support body (52) into a pushed out position in which the support body (52) projects beyond the outer contour of the contact plate (42).

7. A vertebral column implant according to claim 6, **characterised in that** the support body (52) is U-shaped and is displaceable parallel to its limbs (53).

8. A vertebral column implant according to claim 6 or 7, **characterised in that** the support body (52) in the pushed-out position is fixed in this position by a locking means and is thereby secured against being pushed in.

9. A vertebral column implant according to any one of the preceding claims, **characterised in that** the length of the contact plate (42) is approximately twice as great as its width.

10. A vertebral column implant according to any one of the preceding claims, **characterised in that** the contact plate (42) is on its side remote from the vertebral body pivotably supported on an additional contact plate (42) via an articulation (44).

11. A vertebral column implant according to claim 10, **characterised in that** the articulation comprises co-operating ceramic convex bearing surfaces (44).

## Revendications

1. Prothèse vertébrale (1) comportant une plaque d'appui (42) formant une surface d'appui destinée à supporter un corps vertébral, **caractérisée en ce qu'**elle comporte un creux (46), destiné à recevoir au moins un bras de support (47), qui est logé de manière à pouvoir pivoter sur la plaque d'appui (42) autour d'un axe de pivotement (48) disposé perpendiculairement sur la plaque d'appui (42), et **en ce que** ledit au moins un bras de support (47) pivotant est apte à pivoter entre une position pivotée vers l'intérieur, dans laquelle la plaque d'appui (42) recouvre totalement ledit au moins un bras de support (47), et une position pivotée vers l'extérieur, dans laquelle ledit au moins un bras de support (47) s'avance au-delà du contour extérieur de la plaque d'appui (42).

2. Prothèse vertébrale selon la revendication 1, **caractérisée en ce que** l'axe de pivotement (48) du bras de support (47) est disposé au niveau d'un bord longitudinal (45) de la plaque d'appui (42).

3. Prothèse vertébrale selon la revendication 1 ou 2, **caractérisée en ce que** sur chacun des bords latéraux opposés de la plaque d'appui (42) est monté respectivement un bras de support (47).

4. Prothèse vertébrale selon l'une quelconque des revendications précédentes, **caractérisée en ce que** le bras de support (47) dans la position pivotée vers l'extérieur est fixé dans cette position par un élément d'arrêt (50) et, de ce fait, est immobilisé contre tout pivotement vers l'intérieur.

5. Prothèse vertébrale selon la revendication 4, **caractérisée en ce que** l'élément d'arrêt est un ressort à lame (50) s'écartant lors d'un pivotement vers l'extérieur du bras de support (47).

6. Prothèse vertébrale comportant une plaque d'appui (42) formant une surface d'appui destinée à supporter un corps vertébral, **caractérisée en ce qu'**elle comporte un creux (46), destiné à recevoir un corps de support (52), qui est apte à coulisser parallèlement à lui-même dans un guidage et qui peut coulisser entre une position coulissée vers l'intérieur, dans laquelle la plaque d'appui (42) recouvre totalement le corps de support (52), et une position coulissée vers l'extérieur, dans laquelle le corps de support (52) s'avance au-delà du contour extérieur de la plaque d'appui (42).

7. Prothèse vertébrale selon la revendication 6, **caractérisée en ce que** le corps de support (52) est réalisé en forme de U et est apte à coulisser parallèlement à ses branches (53).

8. Prothèse vertébrale selon la revendication 6 ou 7, **caractérisée en ce que** le corps de support (52) dans la position coulissée vers l'extérieur est fixé dans cette position par un élément d'arrêt et, de ce fait, est immobilisé contre tout coulissement vers l'intérieur.

9. Prothèse vertébrale selon l'une quelconque des revendications précédentes, **caractérisée en ce que** la longueur de la plaque d'appui (42) est sensiblement égale au double de sa largeur.

10. Prothèse vertébrale selon l'une quelconque des revendications précédentes, **caractérisée en ce que** la plaque d'appui (42), sur son côté détourné du corps vertébral, prend appui de manière pivotante, par l'intermédiaire d'une articulation (44), sur une plaque d'appui (42) supplémentaire.

11. Prothèse vertébrale selon la revendication 10, **caractérisée en ce que** l'articulation comporte des surfaces d'appui (44) bombées coopérantes en céramique.
